# EUROPÄISCHE PATENTSCHRIFT

(11) **EP 2 257 519 B1**
(45) Veröffentlichungstag und Bekanntmachung des Hinweises auf die Patenterteilung: **31.08.2011**
(21) Anmeldenummer: 09713787.1
(22) Anmeldetag: 26.02.2009
(51) Int. Cl.: C07C 67/08, C07C 67/54, C07C 69/54, C09D 133/06, C09J 133/06

(54) **VERFAHREN ZUR HERSTELLUNG VON (METH)ACRYLATEN VON C10-ALKOHOLGEMISCHEN**
METHOD FOR THE PRODUCTION OF (METH)ACRYLATES OF C10 ALCOHOL MIXTURES
PROCÉDÉ DE PRÉPARATION DE (MÉTH)ACRYLATES DE MÉLANGES D'ALCOOLS C10

(30) Priorität: 27.02.2008 EP 08102070
(43) Veröffentlichungstag der Anmeldung: 08.12.2010
(73) Patentinhaber: BASF SE, 67056 Ludwigshafen (DE)
(72) Erfinder: MEISENBURG, Uwe, 68161 Mannheim (DE); HÖFER, Frank, 67098 Bad Dürkheim (DE); SCHWALM, Reinhold, 67157 Wachenheim (DE); GRUBER, Nick, 68161 Mannheim (DE); KARRER, Lothar, 64319 Pfungstadt (DE)
(86) Internationale Anmeldenummer: PCT/EP2009/052241
(87) Internationale Veröffentlichungsnummer: WO 2009/106550

(56) Entgegenhaltungen:
- DE-A1- 10 063 175
- DE-A1- 10 246 869
- DATABASE WPI Week 199240 Thomson Scientific, London, GB; AN 1992-326706 XP002532352 -& JP 04 230239 A (MITSUBISHI KASEI CORP) 19. August 1992 (1992-08-19)
- ANON.: "Binder for water-thinned coating formulation containing a C10 alkyl (meth) acrylate" IP.COM JOURNAL , 7(5A), 20 (NO. IPCOM000151955D), 24 APR 2007 CODEN: IJPOBX; ISSN: 1533-0001, 2007, XP002532353
- DATABASE WPI Week 199316 Thomson Scientific, London, GB; AN 1993-131248 XP002532354 -& JP 05 070404 A (CHISSO CORP) 23. März 1993 (1993-03-23) in der Anmeldung erwähnt

## Beschreibung

Die vorliegende Erfindung betrifft ein Verfahren zur diskontinuierlichen Herstellung von (Meth)acrylaten von C₁₀-Alkoholgemischen durch Veresterung von (Meth)acrylsäure mit einem Isomerengemisch von C₁₀-Alkoholen aus 2-Propylheptanol als Hauptisomer und mindestens einem der C₁₀-Alkohole 2-Propyl-4-methylhexanol, 2-Propyl-5-methylhexanol, 2-Isopropylheptanol, 2-Isopropyl-4-methylhexanol, 2-Isopropyl-5-methylhexanol und/oder 2-Propyl-4,4-dimethylpentanol.

Der Begriff (Meth)acrylsäure steht in dieser Schrift verkürzend für Methacrylsäure und/oder Acrylsäure, (Meth)acrylsäureester für Methacrylsäureester und/oder Acrylsäureester bzw. (Meth)acrylat für Methacrylat und/oder Acrylat.

Das Isomerengemisch von C₁₀-Alkoholen besteht aus 2-Propylheptanol als Hauptisomer und mindestens einem der C₁₀-Alkohole 2-Propyl-4-methylhexanol, 2-Propyl-5-methylhexanol, 2-Isopropylheptanol, 2-Isopropyl-4-methylhexanol, 2-Isopropyl-5-methylhexanol und/oder 2-Propyl-4,4-dimethylpentanol, wobei diese Verbindungen nachstehend zur Abkürzung als "Propylheptanol-Isomere" bezeichnet werden.

Die auf Basis von (Meth)acrylaten von 2-Propylheptanol und Propylheptanol-Isomeren hergestellten Polymere beziehungsweise Copolymere sind in Form von Polymerdispersionen von großer wirtschaftlicher Bedeutung. Sie finden beispielsweise Anwendung als Klebstoffe, Anstrichmittel oder Textil-, Leder- und Papierhilfsmittel.

Die japanischen Offenlegungsschrift JP 05-070403 offenbart die Umesterung von Methylmethacrylat mit 2-Propylheptanol in einer 14 stündigen Reaktion in Gegenwart von para-Toluolsulfonsäure als Katalysator. Das Umesterungsprodukt wird zur Herstellung von drucksensitiven Klebstoffen verwendet.

In der japanischen Offenlegungsschrift JP 05-070404 wird sowohl die Umesterung von Methylmethacrylat mit 2-Propylheptanol zu 2-Propylheptylmethacrylat als auch die Veresterung von Methacrylsäure mit 2-Propylheptanol beschrieben. Die Produkte werden als Weichmacher in UV-härtbaren Harzen eingesetzt.

Die japanische Offenlegungsschrift JP 04-230239 beschreibt die Herstellung von Acrylsäuredecylestern von Alkoholen mit 10 Kohlenstoffatomen, welche als Hauptkomponente 2-Propylheptanol enthalten. Die Veresterung kann durch Umsetzung von Acrylsäure und z.B. dem Rohisomerengemisch von 2 - Propylheptanol, in Gegenwart von einem sauren Katalysator und einem Polymerisationsinhibitor erfolgen.

Aus der DE 102 46 869 A1 ist ein Verfahren zur kontiniuierlichen Herstellung von (Meth)acrylsäureestern durch säurekatalysierte Veresterung von (Meth)acrylsäure bekannt, wobei bevorzugt 6 bis 12 Kohlenstoffatome enthaltende Alkohole für die Veresterung eingesetzt werden.

Bei den in DE 196 04 253 A1 und DE 196 04 267 A1 offenbarten Verfahren handelt es sich ebenfalls um die kontinuierliche Herstellung von (Meth)acrylsäureestern durch säurekatalysierte Veresterung von (Meth)acrylsäure. Es werden jedoch Alkanole mit 1 bis 8 Kohlenstoffen für die Veresterung eingesetzt.

Aus der DE 100 36 879 A1 ist ein kontinuierliches Verfahren zur Herstellung von Estern der (Meth)acrylsäure durch Veresterung der (Meth)acrylsäure mit C₆-C₁₀-Alkanolen bekannt.

Die diskontinuierliche Herstellung von (Meth)acrylsäureestern durch Veresterung ist in WO 02/055472 A1 beschrieben. Offenbart wird die Veresterung von (Meth)acrylsäure mit höheren ein- oder mehrwertigen Alkoholen, Polyether-, oder Polyesteralkoholen. Die nach diesem Verfahren erhaltenen höheren Ester der (Meth)acrylsäure, die bevorzugt ein Molekulargewicht > 200 g/mol aufweisen, lassen sich nicht mehr durch Destillation reinigen.

Die zuvor zitierte Literatur offenbart ausschließlich (Meth)acrylate von 2-Propylheptanol als C₁₀-Alkoholkomponente, Isomerengemische von 2-Propylheptanol werden nicht beschrieben. Darüber hinaus handelt es sich im Wesentlichen um Verfahren zur Kontinuierlichen Herstellung der (Meth)acrylate.

Aus der deutschen Offenlegungsschrift DE 10 2007 001 540 A1 sind C₁₀/C₆-Estergemische auf Basis von 2-Propylheptanol bekannt, wobei es sich demnach um ein Gemisch aus Estern einer aliphatischen oder aromatischen Di- oder Tricarbonsäure mit einer C₁₀-Alkoholkomponente aus 2-Proplyheptanol und Estern einer aliphatischen oder aromatischen Di- oder Tricarbonsäure mit einer C₆-Alkoholkomponente aus n-Hexanol handelt.

Aufgabe der vorliegenden Erfindung was es daher, ein weiteres, alternatives Verfahren zur Herstellung von (Meth)acrylaten von C₁₀-Alkoholgemischen, die insbesondere 2-Propylheptyl(meth)acrylat als Hauptisomer enthalten, zur Verfügung zu stellen, mit dem die (Meth)acrylate von C₁₀-Alkoholgemischen in hohen Ausbeuten und in hohen Reinheiten erhalten werden. Weiterhin sollen Produkte mit niedrigen Farbzahlen resultieren.

Die Aufgabe wurde gelöst durch ein Verfahren zur diskontinuierlichen Herstellung von (Meth)acrylaten von C₁₀-Alkoholgemischen, durch Umsetzung von (Meth)acrylsäure mit einem Isomerengemisch von C₁₀-Alkoholen aus 2-Propylheptanol als Hauptisomer und mindestens einem der C₁₀-Alkohole 2-Propyl-4-methylhexanol, 2-Propyl-5-methylhexanol, 2-Isopropylheptanol, 2-Isopropyl-4-methylhexanol, 2-Isopropyl-5-methylhexanol und/oder 2-Propyl-4,4-dimethylpentanol, in Gegenwart mindestens eines sauren Katalysators und mindestens eines Polymerisationsinhibitors und in Gegenwart eines Lösungsmittels, das mit Wasser ein Azeotrop bildet, man das Azeotrop abdestilliert und kondensiert, wobei das Kondensat in eine wässrige und eine organische Phase zerfällt, wobei man
a) die Veresterung in einem Reaktor mit Umlaufverdampfer und
b) in Gegenwart eines Lösungsmittels durchführt und
c) das Rohprodukt durch anschließende Reindestillation reinigt.

Nach dem erfindungsgemäßen Verfahren wird ein C₁₀-Alkoholgemisch eingesetzt, das als Hauptisomer 2-Propylheptanol enthält. Unter dem Begriff Hauptisomer im Sinne der vorliegenden Erfindung wird ein Gehalt an 2-Propylheptanol von bis zu 100 Gew.-%, bezogen auf das Gesamtgewicht des C₁₀-Alkoholgemischs, verstanden. Der Gehalt an 2-Propylheptanol beträgt im Allgemeinen mindestens 50 Gew.-%, vorzugsweise 60 bis 98 Gew.-% und besonders bevorzugt 80 bis 95 Gew.-%, insbesondere 85 bis 95 Gew.-%, jeweils bezogen auf das Gesamtgewicht des C₁₀-Alkoholgemischs.

Neben 2-Propylheptanol als Hauptisomer enthält das C₁₀-Alkoholgemisch noch mindestens einen der C₁₀-Alkohole 2-Propyl-4-methylhexanol, 2-Propyl-5-methylhexanol, 2-Isopropylheptanol, 2-Isopropyl-4-methylhexanol, 2-Isopropyl-5-methylhexanol und/oder 2-Propyl-4,4-dimethylpentanol. Diese Verbindungen werden nachstehend zur Abkürzung als "Propylheptanol-Isomere" bezeichnet. Die Anwesenheit anderer Isomere der 2-Propylheptanol-Komponente - beispielsweise von denen zu 2-Propylheptanol isomeren Alkoholen 2-Ethyl-2,4-dimethylhexanol, 2-Ethyl-2-methylheptanol und/oder 2-Ethyl-2,5-dimethylhexanol herrührend - in dem C₁₀-Alkoholgemisch ist möglich, aber wenn überhaupt, sind diese nur in Spuren enthalten.

Zur Herstellung von 2-Propylheptanol und Propylheptanol-Isomeren wird an dieser Stelle auf die deutsche Offenlegungsschrift DE 10 2007 001 540 A1 und die darin zitierte Literatur verwiesen.

Geeignete Mischungen von 2-Propylheptanol mit den Propylheptanol-Isomeren umfassen beispielsweise solche aus 60 bis 98 Gew.-% 2-Propylheptanol, 1 bis 15 Gew.-% 2-Propyl-4-methylhexanol, 0,01 bis 20 Gew.-% 2-Propyl-5-methylhexanol und 0,01 bis 24 Gew.-% 2-Isopropylheptanol, wobei die Summe der Anteile der einzelnen Bestandteile 100 Gew.-% nicht überschreitet. Bevorzugt addieren sich die Anteile der einzelnen Bestandteile zu 100 Gew.-%.

Weitere geeignete Mischungen aus 2-Propylheptanol mit den Propylheptanol-Isomeren umfassen beispielsweise solche aus 75 bis 95 Gew.-% 2-Propylheptanol, 2 bis 15 Gew.-% 2-Propyl-4-methylhexanol, 1 bis 20 Gew.-% 2-Propyl-5-methylhexanol, 0,1 bis 4 Gew.-% 2-Isopropylheptanol, 0,1 bis 2 Gew.-% 2-Isopropyl-4-methylhexanol und 0,1 bis 2 Gew.-% 2-Isopropyl-5-methylhexanol, wobei die Summe der Anteile der einzelnen Bestandteile 100 Gew.-% nicht überschreitet. Bevorzugt addieren sich die Anteile der einzelnen Bestandteile zu 100 Gew.-%.

Bevorzugte Mischungen von 2-Propylheptanol mit den Propylheptanol-Isomeren umfassen solche mit 85 bis 95 Gew.-% 2-Propylheptanol, 5 bis 12 Gew.-% 2-Propyl-4-methylhexanol, 0,1 bis 2 Gew.-% 2-Propyl-5-methylhexanol und 0,01 bis 1 Gew.-% 2-Isopropylheptanol, wobei die Summe der Anteile der einzelnen Bestandteile 100 Gew.-% nicht überschreitet. Bevorzugt addieren sich die Anteile der einzelnen Bestandteile zu 100 Gew.-%.

Bevorzugte Mischungen aus 2-Propylheptanol mit den Propylheptanol-Isomeren umfassen weiterhin solche aus 80 bis 92 Gew.-% 2-Propylheptanol, 6 bis 12 Gew.-% 2-Propyl-4-methylhexanol, 5 bis 13 Gew.-% 2-Propyl-5-methylhexanol, 0,1 bis 2 Gew.-% 2-Isopropylheptanol, 0,1 bis 1 Gew.-% 2-Isopropyl-4-methylhexanol und 0,1 bis 1 Gew.-% 2-Isopropyl-5-methylhexanol, wobei die Summe der Anteile der einzelnen Bestandteile 100 Gew.-% nicht überschreitet. Bevorzugt addieren sich die Anteile der einzelnen Bestandteile zu 100 Gew.-%.

Die Zusammensetzung der (Meth)acrylate von C₁₀-Alkoholgemischen, die nach dem erfindungsgemäßen Verfahren hergestellt werden, entspricht praktisch der Zusammensetzung der zu ihrer Herstellung bei der Veresterung verwendeten Propylheptanol-Isomerengemische.

Die Mischungen aus 2-Propylheptanol mit den Propylheptanol-Isomeren können als Verunreinigungen bedingt durch das Herstellverfahren in Spuren noch n-Pentanol, 2-Methylbutanol und/oder 3-Methylbutanol enthalten. Die Gehalte an diesen Alkoholen liegen im Allgemeinen bei jeweils maximal 0,5 Gew.-%, bezogen auf das Gesamtgewicht des C₁₀-Alkoholgemischs.

Das erfindungsgemäße Verfahren ist vorteilhaft, da ein hoher Veresterungsgrad erreicht wird und hohe Ausbeuten erzielt werden. Weiterhin tritt keine wesentliche Polymerisatbildung bei der Veresterung oder Aufarbeitung auf, und das Endprodukt ist weitgehend farblos.

Das bei der Veresterung entstehende Wasser, das mit dem Lösungsmittel ein Azeotrop bildet, wird über eine dem Reaktor aufgesetzte Kolonne ausgeschleust und kondensiert.

Das anfallende Kondensat (Azeotrop) zerfällt in eine wässrige Phase, die ausgeschleust und vorteilhaft aufgearbeitet (Rückextraktion der enthaltenen Säure) wird und eine Lösungsmittelphase, die als Rücklauf in die Kolonne und gegebenenfalls teilweise in den Reaktor und/oder Verdampfer zurückgeführt wird, wie in der DE 199 41 136 A1 und DE 100 63 175 A1 beschrieben ist.

Eine Rückextraktion der enthaltenen (Meth)acrylsäure erfolgt bevorzugt mit dem verwendeten Lösungsmittel als Extraktionsmittel, beispielsweise mit Cyclohexan bei einer Temperatur zwischen 10 und 40 °C und einem Verhältnis von wässriger Phase zu Extraktionsmittel von 1 : 5 - 30, bevorzugt 1 : 10 - 20. Die im Extraktionsmittel enthaltene Säure kann bevorzugt direkt in die Veresterung geführt werden.

Nach Beendigung der Veresterung wird das heiße Reaktionsgemisch rasch abgekühlt und gegebenenfalls mit Lösungsmittel verdünnt.

Anschließend wird das Lösungsmittel vom Zielester destillativ abgetrennt.

Abschließend wird das Rohprodukt, das den Zielester enthält, destillativ aufgereinigt, so dass ein Endprodukt mit besonders niedriger Farbzahl erhalten wird.

Das erfindungsgemäße Verfahren besteht im Wesentlichen aus folgenden Stufen:

### 1) Veresterung

Die Veresterungsapparatur besteht aus einem Reaktor mit Umlaufverdampfer und einer aufgesetzten Destillationskolonne mit Kondensator und Phasentrenngefäß.

Beim Reaktor kann es sich beispielsweise um einen Reaktor mit Doppelwandheizung oder/und innenliegenden Heizschlangen handeln. Vorzugsweise wird ein Reaktor mit außenliegendem Wärmetauscher und Natur- oder Zwangsumlauf (unter Verwendung einer Pumpe) eingesetzt. Im Falle von Naturumlauf wird der Kreisstrom ohne mechanische Hilfsmittel bewerkstelligt.

Geeignete Umlaufverdampfer sind dem Fachmann bekannt und beispielsweise beschrieben in R. Billet, Verdampfertechnik, HTB-Verlag, Bibliographisches Institut Mannheim, 1965, 53. Beispiele für Umlaufverdampfer sind Rohrbündelwärmetauscher, Plattenwärmetauscher etc.

Selbstverständlich können im Umlauf auch mehrere Wärmetauscher vorhanden sein.

Die Destillationskolonne ist von an sich bekannter Bauart und weist die üblichen Einbauten auf. Als Kolonneneinbauten kommen prinzipiell alle gängigen Einbauten in Betracht, beispielsweise Böden, Packungen und/oder Schüttungen. Von den Böden sind Glockenböden, Siebböden, Ventilböden, Thormannböden und/oder Dual-Flow-Böden bevorzugt, von den Schüttungen sind solche mit Ringen, Wendeln, Sattel körpern oder Geflechten bevorzugt.

In der Regel sind 5 bis 20 theoretische Böden ausreichend.

Der Kondensator und das Trenngefäß sind von herkömmlicher Bauart.

(Meth)acrylsäure und das C₁₀-Alkoholgemisch, das als Hauptisomer 2-Propylheptanol enthält, werden in der Regel in äquivalenten Mengen eingesetzt, es kann aber auch ein Unterschuss oder Überschuss an (Meth)acrylsäure verwendet werden.

Sowohl (Meth)acrylsäure als auch (Meth)acrylsäureester sind polymerisationsfähige Verbindungen. Daher ist bereits im Verfahrensschritt der Veresterung auf eine ausreichende Polymerisationsinhibierung zu achten. Geeignete Polymerisationsinhibitoren werden weiter unten offenbart. Von den dort genannten Stabilisatoren ist für die Veresterung insbesondere Kupfer(II)chlorid geeignet.

Vorzugsweise wird ein Überschuss an (Meth)acrylsäure pro zu veresternde Hydroxygruppe (Äquivalente) von 5 - 100 mol% eingestellt, bevorzugt 5 - 50 mol% und besonders bevorzugt 5 -10 mol%.

Als Vesterungskatalysatoren kommen die üblichen Mineralsäuren und Sulfonsäuren in Frage, vorzugsweise Schwefelsäure, Phosphorsäure, Alkylsulfonsäuren (z.B. Methansulfonsäure, Trifluormethansulfonsäure) und Arylsulfonsäuren (z.B. Benzol-, p-Toluoloder Dodecylbenzolsulfonsäure) oder Gemische davon, aber auch saure Ionenaustauscher oder Zeolithe sind denkbar.

Besonders bevorzugt sind Schwefelsäure, Methansulfonsäure und p-Toluolsulfonsäure oder Gemische davon.

Sie werden in der Regel in einer Menge von 0,1 - 5 Gew.-%, bezogen auf das Veresterungsgemisch, eingesetzt, bevorzugt 0,5 - 5 und besonders bevorzugt 1 - 4 Gew.-%.

Falls erforderlich kann der Veresterungskatalysator aus dem Reaktionsgemisch mit Hilfe eines Ionenaustauschers entfernt werden. der Ionenaustauscher kann dabei direkt in das Reaktionsgemisch gegeben und anschließend abfiltriert oder das Reaktionsgemisch kann über eine Ionenaustauscherschüttung geleitet werden.

Bevorzugt wird der Veresterungskatalysator im Reaktionsgemisch belassen.

Als Lösungsmittel zur azeotropen Entfernung des Reaktionswassers eignen sich vor allem aliphatische, cycloaliphatische und aromatische Kohlenwasserstoffe oder Gemische davon.

Vorzugsweise kommen n-Pentan, n-Hexan, n-Heptan, Cyclohexan, Methylcyclohexan, Benzol, Toluol oder Xylol zur Anwendung. Besonders bevorzugt sind Cyclohexan, Methylcyclohexan und Toluol.

Die eingesetzte Menge beträgt beispielsweise 10 - 200 Gew.-%, vorzugsweise 20 - 100 Gew.-%, besonders bevorzugt 30 -100 Gew.-% bezogen auf die Summe von (Meth)acrylsäure und C₁₀-Alkoholgemisch.

Die Reaktionstemperatur liegt im Allgemeinen bei 60 - 140°C, vorzugsweise 70 - 110 °C, ganz besonders bevorzugt bei 75 - 100°C. Die Anfangstemperatur liegt allgemein unter 100 °C, bevorzugt unter 90 °C und besonders bevorzugt unter 80 °C. In der Regel ist Endtemperatur der Veresterung um 5 - 30°C höher als die Anfangstemperatur. Die Temperatur der Veresterung kann durch Variation der Lösungsmittelkonzentration im Reaktionsgemisch bestimmt und geregelt werden, wie in DE 199 41 136 A1 und DE 100 63 175 A1 beschrieben.

Die Veresterung kann drucklos aber auch bei Überdruck oder Unterdruck durchgeführt werden, vorzugsweise wird bei normalem Druck gearbeitet.

Die Reaktionszeit beträgt in der Regel 30 Minuten bis 10 Stunden, vorzugsweise 1 - 6 und besonders bevorzugt 2 - 4 Stunden.

Die Zugabe der Edukte (Meth)acrylsäure und C₁₀-Alkoholgemisch, das als Hauptisomer 2-Propylheptanol enthält, sowie der sonstigen Komponenten wie Lösungsmittel, Polymerisationsinhibitor(gemisch) und Katalysator kann beliebig erfolgen.

In einer bevorzugten Ausführungsform werden Lösungsmittel und das C₁₀-Alkoholgemisch zumindest teilweise, bevorzugt vollständig, im Reaktor vorgelegt und erhitzt. Sobald der Umlauf in Betrieb ist, können die restlichen Komponenten (Meth)acrylsäure, Polymerisationsinhibitor(gemisch) und Katalysator gemeinsam oder getrennt voneinander zudosiert werden. Die Zudosierung erfolgt in der Regel innerhalb von 0,5 - 5 Stunden kontinuierlich oder portionsweise.

Die einsetzbare (Meth)acrylsäure ist nicht beschränkt und kann im Fall von Roh-(Meth)acrylsäure beispielsweise folgende Komponenten aufweisen:

| | |
|---|---|
| (Meth)acrylsäure | 90 - 99,9 Gew.-% |
| Essigsäure | 0,05 - 3 Gew.-% |
| Propionsäure | 0,01 - 1 Gew.-% |
| Diacrylsäure | 0,01 - 5 Gew.-% |
| Wasser | 0,05 - 5 Gew.-% |
| Aldehyde | 0,01 - 0,3 Gew.-% |
| Inhibitoren | 0,01 - 0,1 Gew.-% |
| Maleinsäure(-anhydrid) | 0,001 - 0,5 Gew.-% |

Die eingesetzte Roh-(Meth)acrylsäure ist in der Regel stabilisiert mit 100 - 600 ppm, bevorzugt mit 200 - 500 ppm eines der unten genannten Polymerisationsinhibitoren, bevorzugt Phenothiazin oder Hydrochinonmonomethylether oder anderen Stabilisatoren, die eine vergleichbare Stabilisierung ermöglichen.

Selbstverständlich kann auch Rein-(Meth)acrylsäure eingesetzt werden mit beispielsweise folgender Reinheit:

| | |
|---|---|
| (Meth)acrylsäure | 99,7 - 99,99 Gew.-% |
| Essigsäure | 50 - 1000 Gew.-ppm |
| Propionsäure | 10 - 500 Gew.-ppm |
| Diacrylsäure | 10 - 500 Gew.-ppm |
| Wasser | 50 - 1000 Gew.-ppm |
| Aldehyde | 1 - 500 Gew.-ppm |
| Inhibitoren | 1 - 300 Gew.-ppm |
| Maleinsäure(-anhydrid) | 1 - 200 Gew.-ppm |

Die eingesetzte Rein-(Meth)acrylsäure ist in der Regel stabilisiert mit 100 - 400 ppm, bevorzugt mit 200 - 300 ppm eines der unten genannten Polymerisationsinhibitoren, bevorzugt Phenothiazin oder Hydrochinonmonomethylether oder anderen Stabilisatoren, die eine vergleichbare Stabilisierung ermöglichen.

Das bei der Reaktion gebildete Wasser wird als Azeotrop mit dem Lösungsmittel kontinuierlich über die dem Reaktor aufgesetzte Kolonne aus dem Reaktionsgemisch entfernt und kondensiert, wobei das Kondensat in eine Wasser- und eine organische Phase zerfällt.

Die wässrige Phase des Kondensats, die in der Regel 0,1 -10 Gew.-% (Meth)acrylsäure enthält, wird abgetrennt und ausgeschleust. Vorteilhafterweise kann die darin enthaltene (Meth)acrylsäure mit einem Extraktionsmittel, beispielsweise mit Cyclohexan, bei einer Temperatur zwischen 10 und 40 °C und einem Verhältnis von wässriger Phase zu Extraktionsmittel von 1 : 5 - 30, bevorzugt 1 : 10 - 20, extrahiert und in die Veresterung zurückgeführt werden.

Die organische Phase kann ganz oder teilweise als Rücklauf in die Kolonne und der ggf. überschüssige Rest kann in den Reaktor zurückgeführt werden. Ein Teil dieser Phase kann bei Verwendung eines Naturumlaufs ggf. zur Unterstützung des Naturumlaufs in den Wärmetauscher des Umlaufkreislaufs des Reaktors eingebracht werden, bevorzugt mindestens 10 Gew.-% der organischen Phase, besonders bevorzugt mindestens 15 Gew.-% und ganz besonders bevorzugt mindestens 20 Gew.-%.

Eine vorteilhafte Variante besteht darin, dass die organische Phase (Lösungsmittelphase) in einen Vorratsbehälter geleitet wird und dass aus diesem Behälter die jeweils erforderliche Lösungsmittelmenge zur Aufrechterhaltung des Rücklaufs, zum Einleiten in den Umlaufverdampfer, sowie als Lösungsmittel für Reaktion und Extraktion entnommen wird.

Zur weiteren Unterstützung des Umlaufs kann ein inertes Gas, bevorzugt ein sauerstoffhaltiges Gas, besonders bevorzugt Luft oder ein Gemisch aus Luft und Stickstoff (Magerluft) in den Umlauf geleitet werden, beispielsweise in Mengen von 0,1 - 1, bevorzugt 0,2 - 0,8 und besonders bevorzugt 0,3 - 0,7 m³/m³h, bezogen auf das Volumen des Reaktionsgemisches.

Der Verlauf der Veresterung kann durch Verfolgung der ausgetragenen Wassermenge und/oder die Abnahme der (Meth)acrylsäurekonzentration im Reaktor verfolgt werden.

Die Reaktion kann beispielsweise beendet werden, sobald 90 % der theoretisch zu erwartenden Wassermenge durch das Lösungsmittel ausgetragen worden sind, bevorzugt bei mindestens 95 % und besonders bevorzugt bei mindestens 98 %.

Nach Beendigung der Veresterung wird das Reaktionsgemisch auf übliche Weise rasch auf eine Temperatur von 10 bis 30 °C abgekühlt, gegebenenfalls durch Zugabe von Lösungsmittel eine Zielesterkonzentration von 60 - 80 % eingestellt.

### 2) Vorwäsche und Neutralisation

Das Reaktionsgemisch wird gegebenenfalls in einem Waschapparat mit Wasser oder einer 5 - 30 Gew.-%igen, bevorzugt 5 - 20, besonders bevorzugt 5 - 15 Gew.-%igen Kochsalz-, Kaliumchlorid-, Ammoniumchlorid-, Natriumsulfat- oder Aluminiumsulfatlösung, bevorzugt Kochsalzlösung behandelt.

Das Mengenverhältnis Reaktionsgemisch : Waschflüssigkeit beträgt in der Regel 1 : 0,1 -1, bevorzugt 1 : 0,2 - 0,8, besonders bevorzugt 1 : 0,3 - 0,7.

Die Wäsche kann beispielsweise in einem Rührbehälter oder in einer anderen herkömmlichen Apparatur, z. B. in einer Kolonne oder Mixer-Settler-Apparatur durchgeführt werden.

Verfahrenstechnisch können für eine Wäsche im erfindungsgemäßen Verfahren alle an sich bekannten Extraktions- und Waschverfahren und -apparate eingesetzt werden, z. B. solche, die in Ullmann's Encyclopedia of Industrial Chemistry, 6th ed, 1999 Electronic Release, Kapitel: Liquid - Liquid Extraction - Apparatus, beschrieben sind. Beispielsweise können dies ein- oder mehrstufige, bevorzugt einstufige Extraktionen, sowie solche in Gleich- oder Gegenstromfahrweise sein.

Die Vorwäsche wird bevorzugt dann eingesetzt, wenn Metallsalze, bevorzugt Kupfer oder Kupfersalze als Inhibitoren (mit)verwendet werden.

Die organische Phase der Vorwäsche, die noch geringe Mengen an Katalysator und die Hauptmenge an überschüssiger (Meth)acrylsäure enthält, wird mit einer 5 - 25, bevorzugt 5 - 20, besonders bevorzugt 5 - 15 Gew.-% wässrigen Lösung einer Base, wie z.B. Natronlauge, Kalilauge, Natriumhydrogencarbonat, Natriumcarbonat, Kaliumhydrogencarbonat, Calciumhydroxid, Ammoniakwasser oder Kaliumcarbonat, der gegebenenfalls 5 - 15 Gew.-% Kochsalz, Kaliumchlorid, Ammoniumchlorid oder Ammoniumsulfat zugesetzt sein können, bevorzugt mit Natronlauge oder Natronlauge-Kochsalz-Lösung neutralisiert.

Die Zugabe der Base erfolgt in einer Weise, dass die Temperatur im Apparat nicht über 35 °C ansteigt, bevorzugt zwischen 20 und 35 °C beträgt und der pH-Wert 10 - 14 beträgt. Die Abfuhr der Neutralisationswärme erfolgt gegebenenfalls durch Kühlung des Behälters mit Hilfe von innenliegenden Kühlschlangen oder über eine Doppelwandkühlung.

Das Mengenverhältnis Reaktionsgemisch : Neutralisationsflüssigkeit beträgt in der Regel 1 : 0,1 - 1, bevorzugt 1 : 0,2 - 0,8, besonders bevorzugt 1 : 0,3 - 0,7.

Hinsichtlich der Apparatur gilt das oben Gesagte.

Optional kann zur Entfernung von Base- oder Salzspuren aus dem neutralisierten Reaktionsgemisch eine Nachwäsche vorteilhaft sein, welche analog zur Vorwäsche durchgeführt werden kann.

### 3) Lösungsmitteldestillation

Das gewaschene Reaktionsgemisch wird mit einer derartigen Menge an Lagerstabilisator, bevorzugt Hydrochinonmonomethylether versetzt, dass nach Abtrennung des Lösungsmittels 100 - 500, bevorzugt 200 - 500 und besonders bevorzugt 200 - 400 ppm davon im Zielester enthalten sind.

Die destillative Abtrennung der Hauptmenge an Lösungsmittel erfolgt beispielsweise in einem Rührkessel mit Doppelwandheizung und/oder innenliegenden Heizschlangen unter vermindertem Druck, beispielsweise bei 20 - 700 mbar, bevorzugt 30 bis 500 und besonders bevorzugt 50 - 150 mbar und einer Temperatur von 40 - 80 °C.

Selbstverständlich kann die Destillation auch in einem Fallfilm- oder Dünnschichtverdampfer erfolgen. Dazu wird das Reaktionsgemisch, bevorzugt mehrmals im Kreislauf, unter vermindertem Druck, beispielsweise bei 20 - 700 mbar, bevorzugt 30 bis 500, besonders bevorzugt 50 - 150 mbar und einer Temperatur von 40 - 80 °C durch den Apparat geführt.

Vorteilhaft kann ein inertes Gas, bevorzugt ein sauerstoffhaltiges Gas, besonders bevorzugt Luft oder ein Gemisch aus Luft und Stickstoff (Magerluft) in den Destillationsapparat eingeleitet werden, beispielsweise 0,1 - 1, bevorzugt 0,2 - 0,8 und besonders bevorzugt 0,3 - 0,7 m³/m³h, bezogen auf das Volumen des Reaktionsgemisches.

Der Restlösungsmittelgehalt im Rückstand beträgt nach der Destillation in der Regel unter 5 Gew.-%, bevorzugt 0,5 - 5 Gew.-%.

Das abgetrennte Lösungsmittel wird kondensiert und bevorzugt wieder verwendet.

### 4) Reindestillation

Erfindungsgemäß wird aus dem unter der Lösungsmitteldestillation anfallenden Sumpf der Zielester in einem weiteren Destillationsschritt als Kopfprodukt isoliert und mit mindestens einem der unten genannten Polymerisationsinhibitoren stabilisiert. Von den dort genannten Stabilisatoren sind für die Reindestillation insbesondere Hydrochinonmonomethylether und Phenothiazin geeignet.

Die für diesen Destillationsschritt verwendbare Rektifikationskolonne ist von bekannter Bauart, wie beispielsweise Füllkörperkolonnen, Packungskolonnen oder Bodenkolonnen, und hat trennwirksame Einbauten (z.B. Glocken-, Sieb- oder Dual-Flow-Böden) oder enthält Schüttungen oder gerichtete Packungen. Diese üblichen Einbauten sind wie in Stufe 1) (Veresterung) beschrieben und weisen bevorzugt 10 bis 20 theoretische Böden auf. Auch Dünnschichtverdampfer kommen in Frage. Verdampfer und Kondensator sind ebenfalls herkömmlicher Bauart (s. Stufe 1, Veresterung).

Bevorzugt wird der Zielester bei einer Sumpftemperatur von 100 - 140 °C, bevorzugt von 110 - 130 °C und einem Kopfdruck von 1 bis 100 mbar, bevorzugt von 1 bis 50 mbar, besonders bevorzugt von 1 bis 10 mbar und insbesondere von 1 bis 5 mbar erhalten.

Zur Stabilisierung kann in den Kondensator eine Lösung von 0,05 - 0,5 % Hydrochinonmonomethylether oder ein anderer ähnlich wirksamer Lagerstabilisator eingesprüht werden, wobei die Menge so gewählt wird, dass das Kondensat eine Lagerstabilisatorkonzentration von 10 - 20 ppm aufweist. Ein Teil des Kondensats, bevorzugt 10 - 20 %, kann der Kolonne wieder als Rücklauf zugeführt werden.

Der anfallende Zielester, der als Hauptisomer 2-Propylheptyl(meth)acrylat enthält, hat entsprechend der gaschromatographischen Analyse eine Reinheit von mindestens 98,5 %, bevorzugt mindestens 99,0 % und besonders bevorzugt mindestens 99,5 %.

Das Sumpfprodukt der Reindestillation, das hauptsächlich aus restlichem Zielester, Michael-Additionsprodukten, Stabilisator und Polymeren besteht, kann in eine Rückstandsdestillation und/oder Rückstandsspaltung geleitet werden.

Selbstverständlich ist es auch möglich, die Destillationseinheiten der Lösungsmitteldestillation (Stufe 3) und die Reindestillation zu vereinigen. In diesem Fall wird der reine Zielester über einen Seitenabzug, bevorzugt gasförmig, im unteren Kolonnenbereich, bevorzugt in der unteren Hälfte, besonders bevorzugt im unteren Drittel, ausgeschleust, kondensiert und wie oben beschrieben stabilisiert.

Die nach dem erfindungsgemäßen Verfahren hergestellten Zielester, die als Hauptisomer 2-Propylheptly(meth)acrylat enthalten, zeichnen sich durch eine hohe Reinheit und eine niedrige APHA-Farbzahl (bestimmt nach DIN-ISO 6271) aus. Bevorzugt beträgt die APHA-Farbzahl unter 50, besonders bevorzugt unter 25 und besonders bevorzugt unter 10.

Überraschenderweise lassen sich mit dem erfindungsgemäßen Verfahren (Meth)acrylate von C₁₀-Alkoholgemischen, die als Hauptisomer 2-Propylheptanol enthalten, durch eine Reindestillation reinigen, obwohl es sich dabei um höhere Acrylate handelt, die nach bekanntem Wissen destillativ nicht oder nur schwer zugänglich sind.

(Meth)acrylsäure und (Meth)acrylsäureester von C₁₀-Alkoholgemischen sind polymerisationsfähige Verbindungen. Daher ist in allen Verfahrensschritten auf eine ausreichende Polymerisationsinhibierung zu achten. Eine unerwünschte Polymerisation ist aufgrund der freiwerdenden großen Wärmemenge sicherheitstechnisch bedenklich.

Daher erfolgen beim erfindungsgemäßen Verfahren sowohl die Veresterungsreaktion als auch die thermischen Trennungen vorzugsweise in Gegenwart von üblichen Mengen an sich bekannter Polymerisationsinhibitoren. In der Regel werden, bezogen auf die α,β-monoethylenisch ungesättigten Monomeren, je Einzelsubstanz von 1 bis 10 000 ppm, bevorzugt von 10 bis 5 000 ppm, besonders bevorzugt von 30 bis 2 500 ppm und insbesondere von 50 bis 1 500 ppm eines geeigneten Stabilisators eingesetzt.

Geeignete Stabilisatoren können beispielsweise N-Oxide (Nitroxyl- oder N-Oxyl-Radikale, also Verbindungen, die wenigstens eine >N-O-Gruppe aufweisen), wie z. B. 4-Hydroxy-2,2,6,6-tetramethylpiperidin-N-oxyl, 4-Oxo-2,2,6,6-tetramethylpiperidin-N-oxyl, 4-Acetoxy-2,2,6,6-tetramethyl-piperidin-N-oxyl, 2,2,6,6-Tetramethylpiperidin-N-oxyl, 4,4',4"-Tris(2,2,6,6-tetramethyl-piperidin-N-oxyl)-phosphit oder 3-Oxo-2,2,5,5-tetramethyl-pyrrolidin-N-oxyl; ein- oder mehrwertige Phenole, die ggf. eine oder mehrere Alkylgruppen aufweisen, wie z. B. Alkylphenole, beispielsweise o-, m- oder p-Kresol (Methylphenol), 2-tert.-Butylphenol, 4-tert.-Butylphenol, 2,4-Di-tert.-butylphenol, 2-Methyl-4-tert.-butylphenol, 2-tert.-Butyl-4-methylphenol, 2,6-tert.-Butyl-4-methylphenol, 4-tert.-Butyl-2,6-dimethylphenol oder 6-tert.-Butyl-2,4-dimethylphenol; Chinone, wie z. B. Hydrochinon, Hydrochinonmonomethylether, 2-Methylhydrochinon oder 2,5-Di-tert.-Butylhydrochinon; Hydroxyphenole, wie beispielsweise Brenzcatechin (1,2-Dihydroxybenzol) oder Benzochinon; Aminophenole, wie z. B. p-Aminophenol; Nitrosophenole, wie z. B. p-Nitrosophenol; Alkoxyphenole, wie beispielsweise 2-Methoxyphenol (Guajacol, Brenzcatechinmonomethylether), 2-Ethoxyphenol, 2-Isopropoxyphenol, 4-Methoxyphenol (Hydrochinonmonomethylether), Mono- oder Di-tert.-Butyl-4-methoxyphenol; Tocipherole, wie z. B. α-Tocopherol sowie 2,3-Dihydro-2,2-dimethyl-7-hydroxybenzofuran (2,2-Dimethyl-7-hydroxycumaran), aromatische Amine, wie z. B. N,N-Diphenylamin oder N-Nitroso-diphenylamin; Phenylendiamine, wie z. B. N,N'-Dialkyl-p-phenylendiamin, wobei die Alkylreste gleich oder verschieden sein können und jeweils unabhängig voneinander aus 1 bis 4 Kohlenstoffatomen bestehen und geradkettig oder verzweigt sein können, wie z. B. N,N'-Dimethyl-p-phenylendiamin oder N,N'-Diethyl-p-phenylendiamin, Hydroxylamine, wie z.B. N,N-Diethylhydroxylamin, Imine, wie z. B. Methylethylimin oder Methylen violett, Sulfonamide, wie z. B. N-Methyl-4-toluolsulfonamid oder N-tert.-Butyl-4-toluolsulfonamid, Oxime, wie Aldoxime, Ketoxime oder Amidoxime, wie z. B. Diethylketoxim, Methylethylketoxim oder Salicyladoxim, phosphorhaltige Verbindungen, wie z. B. Triphenylphosphin, Triphenylphosphit, Triethylphosphit, Hypophsophorige Säure oder Alkylester der Phosphorigen Säuren; schwefelhaltige Verbindungen wie z. B. Diphenylsulfid oder Phenothiazin; Metallsalze, wie Kupfer- oder Mangan-, Cer-, Nickel-, Chromsalze, beispielsweise -chloride, - sulfate, -salicylate, -tosylate, -acrylate oder -acetate, wie z. B. Kupferacetat, Kupfer(II)chlorid, Kupfersalicylat, Cer(III)acetat oder Cer(III)ethylhexanoat, oder Gemische davon sein.

Bevorzugt wird als Polymerisationsinhibitor(gemisch) mindestens eine Verbindung aus der Gruppe Hydrochinon, Hydrochinonmonomethylether, Phenothiazin, 4-Hydroxy-2,2,6,6-tetramethylpiperidin-N-oxyl, 4-Oxo-2,2,6,6-tetramethylpiperidin-N-oxyl, 2-tert.-Butylphenol, 4-tert.-Butylphenol, 2,4-Di-tert.-Butylphenol, 2-tert.-Butyl-4-methylphenol, 6-tert.-Butyl-2,4-dimethylphenol, 2,6-Di-tert.-Butyl-4-methylphenol, 2-Methyl-4-tert.-butylphenol, Hypophosphorige Säure, Kupferacetat, Kupfer(II)chlorid, Kupfersalicylat und Cer(III)acetat.

Ganz besonders bevorzugt wird Phenothiazin und/oder Hydrochinonmonomethylether (MEHQ) als Polymerisationsinhibitor verwendet.

Bevorzugt wird der/das Polymerisationsinhibitor(gemisch) als wässrige Lösung eingesetzt.

Zur weiteren Stützung der Stabilisierung kann ein sauerstoffhaltiges Gas, bevorzugt Luft oder ein Gemisch aus Luft und Stickstoff (Magerluft) anwesend sein.

Im Verfahrensschritt der Veresterung wird das sauerstoffhaltige Gas vorzugsweise in den Sumpfbereich der Kolonne und/oder in einen Umlaufverdampfer eindosiert.

Die erfindungsgemäß hergestellten (Meth)acylate von C₁₀-Alkoholgemischen, die 2-Propylheptanol als Hauptisomer enthalten, finden Anwendung beispielsweise als Monomere oder Comonomere in der Herstellung von Dispersionen, die u.a. als Klebstoffe, Anstrichmittel oder Textil-, Leder- und Papierhilfsmittel eingesetzt werden.

Darüber hinaus können die nach dem erfindungsgemäßen Verfahren hergestellten (Meth)acrylate von C₁₀-Alkoholgemischen, die als Hauptisomer 2-Propylheptanol enthalten, als Comonomer in Polymeren Anwendung finden, die wiederum als Additiv für Brennstofföle und Schmierstoffe und insbesondere als Kaltfließverbesserer in Brennstoffölen eingesetzt werden. Eine derartige Verwendung ist beispielsweise in der europäischen Anmeldung mit dem Aktenzeichen EP 06 124 356.4 offenbart.

Das folgende Beispiel soll die Eigenschaften der Erfindung erläutern, ohne sie aber einzuschränken.

Falls nicht anders angegeben, bedeuten Prozent immer Gewichtsprozent und Teile immer Gewichtsteile.

### Beispiel

In einer Veresterungsapparatur (1 L-4-Halskolben mit Innenthermometer, Rückflusskühler und Wasserabscheider) wurde die Veresterung von Acrylsäure mit 2-Propylheptanol durchgeführt. Dabei wurden 132 ml Cyclohexan, 94,8 g (0,6 mol) 2-Propylheptanol (enthielt ca. 88,3 % 2-Propylheptanol als Hauptisomer, als Nebenisomere waren noch ca. 9,7 %4-Methyl-2-propylhexanol und ca. 1,9 % 5-Methyl-2-propylhexanol enthalten) und 1,5 ml 50 %ige Hypophosphorige Säure vorgelegt und für 2 Stunden bei 60 °C gerührt. Anschließend wurden 3 ml Stabilisatorlösung (1,25 g Hydrochinonmonomethylether (MEHQ) und 3,25 g Hypophosphorige Säure gelöst in 37,5 g Wasser), 0,3 ml 20 %ige Kupfer(II)chloridlösung und 31,8 g (0,66 mol) Acrylsäure (stabilisiert mit 200 ppm MEHQ) zugegeben. Das Gemisch wurde unter Luft-Atmosphäre erhitzt, und bei einer Innentemperatur von 75 °C wurden 2,4 ml 98 %ige Methansulfonsäure zugegeben. Nach 2 Stunden Sieden unter Rückfluss, wobei kontinuierlich Wasser abgetrennt wurde, wurde die Reaktionslösung abgekühlt.

Zu der entstandenen klaren Lösung wurden 60 ml 7,5 %ige Natriumchloridlösung gegeben. Mit 40 ml einer 12,5 %igen Natronlauge wurde ein pH-Wert von 13 eingestellt. Nach dem Ausschütteln wurde die Cyclohexanphase abgetrennt, über Natriumsulfat getrocknet, filtriert und mit 24,5 g (200 ppm) MEHQ versetzt. Anschließend wurde das Lösungsmittel im Vakuum entfernt. Es wurde eine klare Flüssigkeit erhalten.

Man erhielt 2-Propylheptylacrylat in einer Ausbeute von 124 g (97 %) und einer Reinheit > 95 % sowie einer APHA-Farbzahl von 11.

Das Rohprodukt wurde destillativ gereinigt, die Parameter und Ergebnisse der Destillation sind in Tabelle 1 zusammengefasst.

**Tabelle 1: Parameter und Ergebnisse der Destillation**

| Fraktion | Ausbeute [g] | Übergangstemperatur [°C] | Druck [mbar] | APHA-Farbzahl |
|---|---|---|---|---|
| Rohprodukt | 124,0 | -- | -- | 11 |
| Fraktion 1 | 30,8 | 81 - 83 | 0,4 - 0,5 | -- |
| Fraktion 2 | 42,9 | 83 - 85 | 0,5 | - |
| Fraktion 3 | 35,5 | 85 - 86 | 0,5 | - |
| ∑ Fraktionen 1 - 3 | 109,2 | -- | -- | 8 |

Der Zielester 2-Propylheptylacrylat wurde in hoher Reinheit (> 99 %) sowie mit niedriger APHA-Farbzahl erhalten wird.

## Patentansprüche

1. Verfahren zur diskontinuierlichen Herstellung von (Meth)acrylaten von C₁₀-Alkoholgemischen, durch Umsetzung von (Meth)acrylsäure mit einem Isomerengemisch von C₁₀-Alkoholen aus 2-Propylheptanol als Hauptisomer und mindestens einem der C₁₀-Alkohole 2-Propyl-4-methylhexanol, 2-Propyl-5-methylhexanol, 2-Isopropylheptanol, 2-Isopropyl-4-methylhexanol, 2-Isopropyl-5-methylhexanol und/oder 2-Propyl-4,4-dimethylpentanol, in Gegenwart mindestens eines sauren Katalysators und mindestens eines Polymerisationsinhibitors und in Gegenwart eines Lösungsmittels, das mit Wasser ein Azeotrop bildet, man das Azeotrop abdestilliert und kondensiert, wobei das Kondensat in eine wässrige und eine organische Phase zerfällt, **dadurch gekennzeichnet, dass**
a)die Veresterung in einem Reaktor mit Umlaufverdampfer und
b)in Gegenwart eines Lösungsmittels durchführt wird, und
c)dass das Rohprodukt durch anschließende Reindestillation gereinigt wird.

2. Verfahren nach Anspruch 1, **dadurch gekennzeichnet, dass** das Isomerengemisch von C₁₀-Alkoholen aus 60 bis 98 Gew.-% 2-Propylheptanol, 1 bis 15 Gew.-% 2-Propyl-4-methylhexanol, 0,01 bis 20 Gew.-% 2-Propyl-5-methylhexanol und 0,01 bis 24 Gew.-% 2-Isopropylheptanol besteht, wobei die Summe der Anteile der einzelnen Bestandteile 100 Gew.-% nicht überschreitet.

3. Verfahren nach Anspruch 1, **dadurch gekennzeichnet, dass** das Isomerengemisch von C₁₀-Alkoholen aus 75 bis 95 Gew.-% 2-Propylheptanol, 2 bis 15 Gew.-% 2-Propyl-4-methylhexanol, 1 bis 20 Gew.-% 2-Propyl-5-methylhexanol, 0,1 bis 4 Gew.-% 2-Isopropylheptanol, 0,1 bis 2 Gew.-% 2-Isopropyl-4-methylhexanol und 0,1 bis 2 Gew.-% 2-Isopropyl-5-methylhexanol besteht, wobei die Summe der Anteile der einzelnen Bestandteile 100 Gew.% nicht überschreitet.

4. Verfahren nach einem der vorstehenden Ansprüche, **dadurch gekennzeichnet, dass** die Reindestillation bei einer Sumpftemperatur von 100 - 140 °C und einem Kopfdruck von 1 bis 100 mbar durchgeführt wird.

5. Verfahren nach einem der vorstehenden Ansprüche, **dadurch gekennzeichnet, dass** die Reindestillation bei einer Sumpftemperatur von 110 - 130 °C und einem Kopfdruck von 1 bis 50 mbar durchgeführt wird.

6. Verfahren nach einem der vorstehenden Ansprüche, **dadurch gekennzeichnet, dass** bei der Reindestillation ein Teil des Kondensats der Kolonne wieder als Rücklauf zugeführt wird.

7. Verfahren nach einem der vorstehenden Ansprüche, **dadurch gekennzeichnet, dass** man als Polymerisationsinhibitor(gemisch) mindestens eine Verbindung aus der Gruppe Hydrochinon, Hydrochinonmonomethylether, Phenothiazin, 4-Hydroxy-2,2,6,6-tetramethylpiperidin-N-oxyl, 4-Oxo-2,2,6,6-tetramethylpiperidin-N-oxyl, 2-tert.-Butylphenol, 4-tert.-Butylphenol, 2,4-Di-tert.-Butylphenol, 2-tert.-Butyl-4-methylphenol, 6-tert.-Butyl-2,4-dimethylphenol, 2,6-Di-tert.-Butyl-4-methylphenol, 2-Methyl-4-tert.-butylphenol, Hypophosphorige Säure, Kupferacetat, Kupfer(II)chlorid, Kupfersalicylat und Cer(III)acetat einsetzt.

## Claims

1. A process for batchwise preparation of (meth)acrylates of C₁₀-alcohol mixtures, by reacting (meth)acrylic acid with an isomer mixture of C₁₀-alcohols composed of 2-propylheptanol as the main isomer and at least one of the C₁₀-alcohols 2-propyl-4-methylhexanol, 2-propyl-5-methylhexanol, 2-isopropylheptanol, 2-isopropyl-4-methylhexanol, 2-isopropyl-5-methylhexanol and/or 2-propyl-4,4-dimethylpentanol, in the presence of at least one acidic catalyst and of at least one polymerization inhibitor and in the presence of a solvent which forms an azeotrope with water, the azeotrope is distilled off and condensed, and the condensate splits into an aqueous phase and an organic phase, which comprises
a) performing the esterification in a reactor with a circulation evaporator and
b) in the presence of a solvent, and
c) purifying the crude product by subsequent purifying distillation.

2. The process according to claim 1, wherein the isomer mixture of C₁₀-alcohols consists of from 60 to 98% by weight of 2-propylheptanol, from 1 to 15% by weight of 2-propyl-4-methylhexanol, from 0.01 to 20% by weight of 2-propyl-5-methylhexanol and from 0.01 to 24% by weight of 2-isopropylheptanol, where the sum of the proportions of the individual constituents does not exceed 100% by weight.

3. The process according to claim 1, wherein the isomer mixture of C₁₀-alcohols consists of from 75 to 95% by weight of 2-propylheptanol, from 2 to 15% by weight of 2-propyl-4-methylhexanol, from 1 to 20% by weight of 2-propyl-5-methylhexanol, from 0.1 to 4% by weight of 2-isopropylheptanol, from 0.1 to 2% by weight of 2-isopropyl-4-methylhexanol and from 0.1 to 2% by weight of 2-isopropyl-5-methylhexanol, where the sum of the proportions of the individual constituents does not exceed 100% by weight.

4. The process according to any of the preceding claims, wherein the purifying distillation is carried out at a bottom temperature of 100 - 140°C and a top pressure of from 1 to 100 mbar.

5. The process according to any of the preceding claims, wherein the purifying distillation is carried out at a bottom temperature of 110 -130°C and a top pressure of from 1 to 50 mbar.

6. The process according to any of the preceding claims, wherein, in the purifying distillation, a portion of the condensate is fed back to the column as reflux.

7. The process according to any of the preceding claims, wherein the polymerization inhibitor (mixture) used is at least one compound from the group of hydroquinone, hydroquinone monomethyl ether, phenothiazine, 4-hydroxy-2,2,6,6-tetramethylpiperidine N-oxyl, 4-oxo-2,2,6,6-tetramethylpiperidine N-oxyl, 2-tert-butylphenol, 4-tert-butylphenol, 2,4-di-tert-butylphenol, 2-tert-butyl-4-methylphenol, 6-tert-butyl-2,4-dimethylphenol, 2,6-di-tert-butyl-4-methylphenol, 2-methyl-4-tert-butylphenol, hypophosphorous acid, copper acetate, copper(II) chloride, copper salicylate and cerium(III) acetate.

## Revendications

1. Procédé pour la préparation discontinue de (méth)acrylates de mélanges d'alcools en C₁₀ par transformation d'acide (méth)acrylique avec un mélange d'isomères d'alcools en C₁₀ constitué par du 2-propylheptanol comme isomère principal et au moins un des alcools en C₁₀ parmi le 2-propyl-4-méthylhexanol, le 2-propyl-5-méthylhexanol, le 2-isopropylheptanol, le 2-isopropyl-4-méthylhexanol, le 2-isopropyl-5-méthylhexanol et/ou le 2-propyl-4,4-diméthylpentanol, en présence d'au moins un catalyseur acide et d'au moins un inhibiteur de polymérisation et en présence d'un solvant, qui forme un azéotrope avec de l'eau, on élimine l'azéotrope par distillation et on condense, le condensat se décomposant en une phase aqueuse et une phase organique, **caractérisé en ce que**
a) l'estérification est réalisée dans un réacteur avec un évaporateur à circulation et
b) en présence d'un solvant, et
c) le produit brut est purifié par distillation pure consécutive.

2. Procédé selon la revendication 1, **caractérisé en ce que** le mélange d'isomères d'alcools en C₁₀ est constitué par 60 à 98% en poids de 2-propylheptanol, 1 à 15% en poids de 2-propyl-4-méthylhexanol, 0,01 à 20% en poids de 2-propyl-5-méthylhexanol et 0,01 à 24% en poids de 2-isopropylheptanol, où la somme des proportions des différents constituants ne dépasse pas 100% en poids.

3. Procédé selon la revendication 1, **caractérisé en ce que** le mélange d'isomères d'alcools en C₁₀ est constitué par 75 à 95% en poids de 2-propylheptanol, 2 à 15% en poids de 2-propyl-4-méthylhexanol, 1 à 20% en poids de 2-propyl-5-méthylhexanol, 0,1 à 4% en poids de 2-isopropylheptanol, 0,1 à 2% en poids de 2-isopropyl-4-méthylhexanol et 0,1 à 2% en poids de 2-isopropyl-5-méthylhexanol, où la somme des proportions des différents constituants ne dépasse pas 100% en poids.

4. Procédé selon l'une quelconque des revendications précédentes, **caractérisé en ce que** la distillation pure est réalisée à une température du fond de 100-140°C et à une pression de tête de 1 à 100 bars.

5. Procédé selon l'une quelconque des revendications précédentes, **caractérisé en ce que** la distillation pure est réalisée à une température du fond de 110-130°C et à une pression de tête de 1 à 50 bars.

6. Procédé selon l'une quelconque des revendications précédentes, **caractérisé en ce que** lors de la distillation pure, une partie du condensat de la colonne est à nouveau alimentée comme reflux.

7. Procédé selon l'une quelconque des revendications précédentes, **caractérisé en ce qu'**on utilise, comme (mélange de) inhibiteur(s) de polymérisation, au moins un composé du groupe formé par l'hydroquinone, l'hydroquinonemonométhyléther, la phénothiazine, le 4-hydroxy-2,2,6,6-tétraméthylpipéridine-N-oxyle, le 4-oxo-2,2,6,6-tétraméthylpipéridin-N-oxyle, le 2-tert-butylphénol, le 4-tert-butylphénol, le 2,4-di-tert-butylphénol, le 2-tert-butyl-4-méthylphénol, le 6-tert-butyl-2,4-diméthylphénol, le 2,6-di-tert-butyl-4-méthylphénol, le 2-méthyl-4-tert-butylphénol, l'acide hypophosphoreux, l'acétate de cuivre, le chlorure de cuivre (II), le salicylate de cuivre et l'acétate de cérium (III).
